# EUROPEAN PATENT APPLICATION

(11) **EP 4 817 676 A1**
(43) Date of publication of application: **07.10.2026**
(21) Application number: 24898300.9
(22) Date of filing: 18.11.2024
(51) Int. Cl.: A61N 1/40, A61N 1/36, A61N 1/32, A61N 1/06

(54) **BIPOLAR RF ENERGY GENERATION DEVICE**

(30) Priority: 28.11.2023 KR 20230168291
(71) Applicant: CLASSYS INC., Seoul 06220 (KR)
(72) Inventor: PARK, Sung Jun, Seongnam-si Gyeonggi-do 13581 (KR); KIM, Jin Yong, Guri-si Gyeonggi-do 11902 (KR); KIM, Hye Ri, Seoul 05038 (KR)
(74) Representative: Witte, Weller & Partner Patentanwälte mbB
(86) International application number: PCT/KR2024/096610
(87) International publication number: WO 2025/116708

(57) **Abstract**

The present invention relates to a bipolar RF energy generation device. According to one embodiment, the bipolar RF energy generation device comprises: a cartridge housing unit having one side that comes in contact with the skin; and at least three RF electrode units positioned on one surface of the cartridge housing unit so as to be spaced apart from one another, thereby coming in contact with the skin, wherein an electric power source is applied to the two RF electrode units, each of the interval of the RF electrode units to which the electric power source is applied and the frequency of RF energy are adjusted such that the skin depth to which the RF energy is transmitted can be variously adjusted, and uniform energy is transmitted for each skin depth so that, in a bipolar method, a procedure effect can be enhanced and a procedure type can be diversified.

## Description

### Technical Field

The present disclosure relates to a bipolar RF energy generation device and, more particularly, to a bipolar RF energy generation device that adjusts a distance between RF electrodes and delivers uniform energy according to skin depth by using multiple frequencies.

### Background Art

In general, an RF energy generation device used for cosmetic purposes for skin is a device that performs a procedure by heating the skin and subcutaneous tissue by using a radio frequency (RF) electrode.

The RF energy generation device is used to promote recovery skin after a cryolipolysis procedure by bringing an RF electrode into contact with the skin and then delivering RF energy to the skin to increase a temperature of the skin, and is also used in skin treatment for causing contraction of collagen or inducing a wound healing response by heating the skin and subcutaneous tissue.

In recent years, with increasing interest in skin aesthetics, the bipolar RF energy generation device has been variously used for skin remodeling/resurfacing, wrinkle removal, and treatment of sebaceous glands, hair follicle adipose tissue, and spider veins through contraction of collagen in a lower dermis or a wound healing response.

RF energy generation devices mainly include a monopolar-type device having one electrode that contacts the skin, a bipolar-type device having two electrodes that contact the skin, and the like.

A bipolar RF energy generation device uses a pair of RF electrodes contacting the skin, wherein one RF electrode serves as a positive electrode (+) and the other RF electrode serves as a negative electrode (-), so that RF energy, that is, high-frequency energy, emitted from the positive electrode (+) flows into the negative electrode (-), thereby delivering the RF energy into the skin.

A conventional bipolar RF energy generation device has a structure in which a distance between a pair of RF electrodes contacting the skin is fixed. Therefore, a delivery depth of RF energy in the skin can be controlled only by controlling a frequency of the RF energy, thereby limiting the delivery depth of RF energy in the skin.

In addition, since the conventional bipolar RF energy generation device controls the delivery depth of RF energy only by controlling the frequency of the RF energy, there is a problem in that high-frequency RF energy cannot deeply penetrate into the skin.

That is, the conventional bipolar RF energy generation device has problems in that a delivery depth of RF energy in the skin is limited, and RF energy having a high frequency cannot deeply penetrate into the skin, so that treatment effects obtainable during a procedure are limited and the device is difficult to apply to various procedures.

A prior patent related to the present disclosure is Korean Patent No. 10-0993491, entitled "High-Frequency Output Device," registered on November 4, 2010.

### Disclosure

### Technical Problem

The objective of the present disclosure is to provide a bipolar RF energy generation device capable of adjusting a distance between RF electrodes, delivering uniform energy according to skin depth by using multiple frequencies, and variously controlling a delivery depth of RF energy in the skin.

### Technical Solution

In order to accomplish the above objectives, according to an embodiment of the present disclosure, a bipolar RF energy generation device includes: a cartridge housing portion having one surface configured to contact skin; at least three RF electrode portions positioned on the one surface of the cartridge housing portion to be spaced apart from one another and configured to contact the skin; and a power control portion configured to apply electrical power to two of the RF electrode portions and to control a depth of RF energy delivered into the skin by varying a distance between the two RF electrode portions to which the electrical power is applied.

In the present disclosure, the power control portion may be configured to apply electrical power to two RF electrode portions selected from among the three RF electrode portions and spaced apart from each other by a first distance, or to apply electrical power to two RF electrode portions selected from among the three RF electrode portions and spaced apart from each other by a second distance longer than the first distance.

In the present disclosure, the at least three RF electrode portions may include: a reference RF electrode portion; and a plurality of selectable RF electrode portions spaced apart from the reference RF electrode portion by different distances, wherein, when applying electrical power to two electrodes, the power control portion may be configured to constantly apply electrical power to the reference RF electrode portion, connect any one of the plurality of selectable RF electrode portions to a power supply line through a power application switch portion, and apply electrical power to the connected selectable RF electrode portion.

In the present disclosure, the at least three RF electrode portions may be arranged in a line to be spaced apart from one another.

In the present disclosure, the at least three RF electrode portions may include a first RF electrode portion, a second RF electrode portion, a third RF electrode portion, and a fourth RF electrode portion positioned in a line, and the power control portion may be configured to apply electrical power to the first RF electrode portion and the fourth RF electrode portion positioned at opposite ends, or to apply electrical power to the second RF electrode portion and the third RF electrode portion positioned between the first RF electrode portion and the fourth RF electrode portion.

In the present disclosure, the at least three RF electrode portions may be positioned to be spaced apart from one another at different intervals, and the power control portion may be configured to select two RF electrode portions from among the three RF electrode portions and apply electrical power thereto.

In the present disclosure, the RF electrode portions may include an even number of at least four RF electrode portions spaced apart from one another, and the power control portion may be configured to simultaneously apply electrical power to at least two pairs of the RF electrode portions.

In the present disclosure, the power control portion may be configured to make a voltage of electrical power applied to one pair of the RF electrode portions different from a voltage of electrical power applied to another pair of the RF electrode portions.

In the present disclosure, the at least three RF electrode portions may include: at least one fixed electrode portion fixed to the one surface of the cartridge housing portion; and a plurality of movable electrode portions movably positioned at one side of the fixed electrode portion, wherein the plurality of movable electrode portions may be moved toward one side of the fixed electrode portion by an electrode movement portion provided in the cartridge housing portion.

In the present disclosure, the fixed electrode portion may include a first fixed electrode portion and a second fixed electrode portion positioned at one side of the first fixed electrode portion to be spaced apart therefrom, and the movable electrode portions may include a first movable electrode portion and a second movable electrode portion movably positioned between the first fixed electrode portion and the second fixed electrode portion.

In the present disclosure, the electrode movement portion may include: a movement screw rotatably positioned in the cartridge housing portion and passing through the first movable electrode portion and the second movable electrode portion, the first movable electrode portion and the second movable electrode portion being screw-coupled to the movement screw in opposite screw directions; and a screw rotation motor configured to rotate the movement screw.

In the present disclosure, the cartridge housing portion may include, on one surface thereof, an electrode-mounting protrusion provided with the first fixed electrode portion and the second fixed electrode portion, wherein an electrode movement space in which the first movable electrode portion and the second movable electrode portion are movably positioned may be provided within the electrode-mounting protrusion.

### Advantageous Effects

According to the present disclosure, electrical power is selectively applied to two RF electrode portions among at least three RF electrode portions, and a distance between the RF electrode portions to which the electrical power is applied and a frequency of RF energy are respectively controlled, thereby variously controlling a delivery depth of the RF energy in the skin and delivering uniform energy according to skin depth, so that effects of a procedure can be improved in a bipolar method and types of procedures can be diversified.

### Description of Drawings

FIGS. 1 and 2 are schematic diagrams illustrating one embodiment of a bipolar RF energy generation device according to the present disclosure.
FIG. 3 is a schematic diagram illustrating another embodiment of the bipolar RF energy generation device according to the present disclosure.
FIG. 4 is a plan view illustrating still another embodiment of the bipolar RF energy generation device according to the present disclosure.
FIG. 5 is a schematic diagram illustrating yet another embodiment of the bipolar RF energy generation device according to the present disclosure.

### * Description of the Reference Numerals in the Drawings

10 : Skin
100 : Cartridge housing portion 110 : Electrode-mounting protrusion
120 : Electrode movement space 200 : Reference RF electrode portion
201 : Selectable RF electrode portion 210 : First RF electrode portion
220 : Second RF electrode portion 230 : Third RF electrode portion
240 : Fourth RF electrode portion 250 : First fixed electrode portion
260 : Second fixed electrode portion 270 : First movable electrode portion
280 : Second movable electrode portion 300 : Power control portion
310 : Power application switch portion 320 : Impedance sensing portion
400 : Electrode movement portion 410 : Movement screw
420 : Screw rotation motor

### Best Mode

Hereinafter, preferred embodiments of the present disclosure will be described in detail with reference to the accompanying drawings. However, the technical spirit of the present disclosure is not limited to the embodiments described herein and may be embodied in other forms. Rather, the embodiments introduced herein are provided so that the disclosed content may be thorough and complete and so that the spirit of the present disclosure may be sufficiently conveyed to those skilled in the art.

In the present specification, when an element is referred to as being on another element, it means that the element may be directly formed on the other element, or a third element may be interposed therebetween. In addition, in the drawings, thicknesses of shapes and regions are exaggerated for effective description of technical content.

In addition, although terms such as first, second, third are used in various embodiments of the present specification to describe various elements, these elements should not be limited by such terms. These terms are used merely to distinguish one element from another element. Accordingly, an element referred to as a first element in one embodiment may be referred to as a second element in another embodiment. Each embodiment described and illustrated herein also includes a complementary embodiment thereof. In addition, in the present specification, "and/or" is used to mean including at least one of elements listed before and after the term.

In the specification, a singular expression includes a plural expression unless the context clearly indicates otherwise. In addition, terms such as "includes" or "have" are intended to specify the presence of features, numbers, steps, elements, or combinations thereof described in the specification, and should not be understood as excluding the presence or addition possibility of one or more other features, numbers, steps, elements, or combinations thereof. In addition, in the present specification, "connection" is used to mean including both indirect connection of a plurality of elements and direct connection thereof.

In addition, in describing the present disclosure below, when it is determined that a detailed description of related known functions or configurations may unnecessarily obscure the gist of the present disclosure, the detailed description thereof will be omitted.

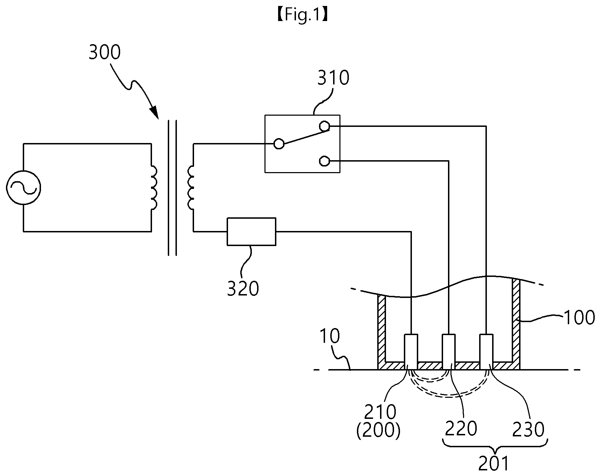

FIG. 1 is a schematic diagram illustrating one embodiment of a bipolar RF energy generation device according to the present disclosure, and the one embodiment of the bipolar RF energy generation device according to the present disclosure will be described below in detail with reference to FIG. 1.

According to the one embodiment of the present disclosure, the bipolar RF energy generation device includes a cartridge housing portion (100) having one surface configured to contact skin (10), wherein a plurality of RF electrode portions (210, 220, 230) configured to contact the skin (10) and deliver RF energy to the skin (10) are provided on the one surface.

The RF electrode portions (210, 220, 230) have a preset number of uses, and when the RF electrode portions (210, 220, 230) reach the preset number of uses, that is, reach the end of their service life, the cartridge housing portion (100) is replaced with an unused new cartridge housing portion (100).

Although not illustrated, the cartridge housing portion (100) may, for example, have a structure in which the cartridge housing portion (100) is connected to a control body portion (not shown) configured to control operation of the RF electrode portions in an RF skin-beauty device used for a skin procedure and is detachably mounted to a handpiece that an operator holds and uses by hand.

It should be noted that the cartridge housing portion (100) may be implemented with various modifications as a cartridge coupling structure detachably coupled to a handpiece in a known RF skin-beauty device, and thus a more detailed description thereof will be omitted.

At least three RF electrode portions (210, 220, 230) positioned to be spaced apart from one another are provided on one surface of the cartridge housing portion (100).

The three RF electrode portions (210, 220, 230) are connected to a power control portion (300) and selectively receive electrical power in a state of being in contact with skin (10) of a patient, thereby generating RF energy.

The power control portion (300) may apply electrical power to two RF electrode portions, which are in contact with the skin (10), among the three RF electrode portions (210, 220, 230), thereby delivering RF energy to the skin (10).

In addition, the power control portion (300) controls a depth of RF energy delivered into the skin (10) by varying a distance between two RF electrode portions to which electrical power is applied among the three RF electrode portions (210, 220, 230).

When RF energy is generated in a bipolar method, a delivery depth of the RF energy in the skin (10) varies according to a distance between two RF electrode portions to which power is applied.

As a distance between the two RF electrode portions (210, 220) decreases, a penetration depth of RF energy delivered into the skin (10) decreases, and as a distance between the two RF electrode portions (220, 230) increases, the penetration depth of RF energy delivered into the skin (10) increases.

In addition, when RF energy is generated in a bipolar method, a delivery depth of the RF energy in the skin (10) varies according to a frequency of the RF energy applied to two RF electrode portions.

As the frequency of the RF energy applied to the two RF electrode portions increases, a penetration depth of RF energy delivered into the skin (10) decreases, and as the frequency of the RF energy applied to the two RF electrode portions decreases, the penetration depth of RF energy delivered into the skin (10) increases.

The power control portion (300) may apply electrical power to two RF electrode portions (210, 220) spaced apart from each other by a first distance among the three RF electrode portions (210, 220, 230), or may apply electrical power to two RF electrode portions (220, 230) spaced apart from each other by a second distance longer than the first distance among the three RF electrode portions (210, 220, 230).

The power control portion (300) may apply electrical power to the two RF electrode portions (210, 220) spaced apart from each other by the first distance to decrease a penetration depth of RF energy, and may apply electrical power to the two RF electrode portions (220, 230) spaced apart from each other by the second distance to increase the penetration depth of RF energy.

As one example, the at least three RF electrode portions (210, 220, 230) include a first RF electrode portion (210), a second RF electrode portion (220), and a third RF electrode portion (230) arranged in a line to be spaced apart from one another.

The first RF electrode portion (210), the second RF electrode portion (220), and the third RF electrode portion (230) are exposed on a plane and simultaneously contact the skin (10) during a procedure.

The first RF electrode portion (210), the second RF electrode portion (220), and the third RF electrode portion (230) are arranged in a line on one surface of the cartridge housing portion (100), and are arranged to be spaced apart from one another in the order of the first RF electrode portion (210), the second RF electrode portion (220), and the third RF electrode portion (230) from the left.

That is, the second RF electrode portion (220) is positioned at one side of the first RF electrode portion (210) to be spaced apart therefrom, and the third RF electrode portion (230) is positioned at one side of the second RF electrode portion (220) to be spaced apart therefrom.

When applying electrical power to two RF electrode portions among the first RF electrode portion (210), the second RF electrode portion (220), and the third RF electrode portion (230), the power control portion (300) may constantly apply electrical power to the first RF electrode portion (210), and may apply electrical power to any one of the second RF electrode portion (220) and the third RF electrode portion (230), thereby controlling a penetration depth of RF energy.

The power control portion (300) may include a power application switch portion (310) configured to apply electrical power to any one of the second RF electrode portion (220) and the third RF electrode portion (230), thereby connecting any one of the second RF electrode portion (220) and the third RF electrode portion (230) to a power supply line.

The power control portion (300) may apply electrical power to the first RF electrode portion (210) and the second RF electrode portion (220) to deliver RF energy to a first depth in the skin (10), or may apply electrical power to the first RF electrode portion (210) and the third RF electrode portion (230) to deliver RF energy to a second depth deeper than the first depth in the skin (10), thereby controlling a penetration depth of RF energy to the first depth or the second depth.

In addition, an impedance sensing portion (320) configured to receive impedance feedback from the first RF electrode portion (210) is positioned on a power line connecting the power control portion (300) and the first RF electrode portion (210), and the power control portion (300) receives the impedance feedback from the first RF electrode portion (210) and applies electrical power to any one of the second RF electrode portion (220) and the third RF electrode portion (230), thereby controlling a penetration depth of RF energy.

The power control portion (300) constantly applies electrical power to the first RF electrode portion (210), applies electrical power to any one of the second RF electrode portion (220) and the third RF electrode portion (230), and controls RF energy delivered from the two RF electrode portions, that is, an intensity of an electric field, thereby more precisely and variously controlling a depth to which the RF energy is delivered into the skin (10).

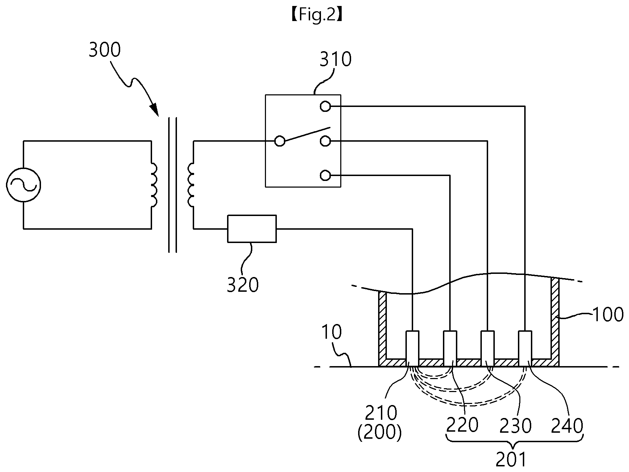

FIG. 2 illustrates, as one embodiment of the bipolar RF energy generation device according to the present disclosure, an example including four RF electrode portions (210, 220, 230, 240), wherein the example includes a first RF electrode portion (210), a second RF electrode portion (220), a third RF electrode portion (230), and a fourth RF electrode portion (240) positioned in a line.

The first RF electrode portion (210), the second RF electrode portion (220), the third RF electrode portion (230), and the fourth RF electrode portion (240) are arranged in a line on one surface of the cartridge housing portion (100), and are arranged to be spaced apart from one another in the order of the first RF electrode portion (210), the second RF electrode portion (220), the third RF electrode portion (230), and the fourth RF electrode portion (240) from the left.

When applying electrical power to two RF electrode portions among the first RF electrode portion (210), the second RF electrode portion (220), the third RF electrode portion (230), and the fourth RF electrode portion (240), the power control portion (300) may constantly apply electrical power to the first RF electrode portion (210), and may apply electrical power to any one of the second RF electrode portion (220), the third RF electrode portion (230), and the fourth RF electrode portion (240), thereby controlling a penetration depth of RF energy.

The power control portion (300) may include a power application switch portion (310) configured to apply electrical power to any one of the second RF electrode portion (220), the third RF electrode portion (230), and the fourth RF electrode portion (240), thereby connecting any one of the second RF electrode portion (220), the third RF electrode portion (230), and the fourth RF electrode portion (240) to a power supply line.

The power control portion (300) applies electrical power to the first RF electrode portion (210) and the second RF electrode portion (220) to deliver RF energy to a first depth in the skin (10), applies electrical power to the first RF electrode portion (210) and the third RF electrode portion (230) to deliver RF energy to a second depth deeper than the first depth in the skin (10), or applies electrical power to the first RF electrode portion (210) and the third RF electrode portion (230) to deliver RF energy to a third depth deeper than the second depth in the skin (10).

The impedance sensing portion (320) configured to receive impedance feedback from the first RF electrode portion (210) is positioned on a power line connecting the power control portion (300) and the first RF electrode portion (210), and the power control portion (300) receives the impedance feedback from the first RF electrode portion (210) and applies electrical power to any one of the second RF electrode portion (220), the third RF electrode portion (230), and the fourth RF electrode portion (240), thereby controlling a penetration depth of RF energy.

The power control portion (300) constantly applies electrical power to the first RF electrode portion (210), applies electrical power to any one of the second RF electrode portion (220), the third RF electrode portion (230), and the fourth RF electrode portion (240), and controls RF energy delivered from the two RF electrode portions, that is, an intensity of an electric field, thereby more precisely and variously controlling a depth to which the RF energy is delivered into the skin (10).

Referring to FIGS. 1 and 2, the at least three RF electrode portions (210, 220, 230, 240) include a reference RF electrode portion (200) and a plurality of selectable RF electrode portions (201) spaced apart from the reference RF electrode portion (200) by different distances, and when electrical power is applied to two RF electrode portions, the power control portion (300) constantly applies electrical power to the reference RF electrode portion (200), connects any one of the plurality of selectable RF electrode portions (201) to a power supply line through the power application switch portion (310), and applies electrical power to the connected selectable RF electrode portion (201), thereby adjusting a distance between the two RF electrode portions to which the electrical power is applied. As the distance between the two RF electrode portions to which the electrical power is applied decreases, a penetration depth of the RF energy into the skin (10) decreases, and as the distance between the two RF electrode portions increases, the penetration depth of the RF energy into the skin (10) increases.

In addition, the impedance sensing portion (320) configured to receive impedance feedback from the reference RF electrode portion (200) is positioned on a power line connecting the power control portion (300) and the reference RF electrode portion (200), and the power control portion (300) receives the impedance feedback from the reference RF electrode portion (200) and applies electrical power to any one of the plurality of selectable RF electrode portions (201), thereby controlling a penetration depth of RF energy.

In addition, when electrical power is applied to two RF electrode portions, the power control portion (300) constantly applies electrical power to the reference RF electrode portion (200), applies electrical power to any one selectable RF electrode portion (201) among the plurality of selectable RF electrode portions (201) to adjust a distance between the two RF electrode portions, and controls RF energy delivered from the two RF electrode portions, that is, an intensity of an electric field, thereby more precisely and variously controlling a depth to which the RF energy is delivered into the skin (10).

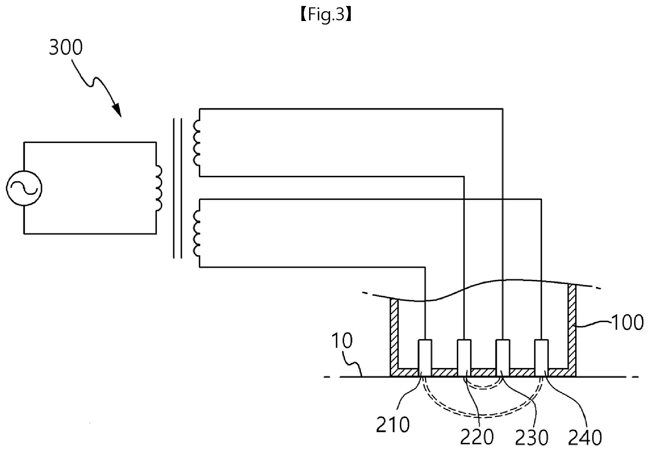

Meanwhile, FIG. 3 is a schematic diagram illustrating another embodiment of the bipolar RF energy generation device according to the present disclosure. Referring to FIG. 3, at least three RF electrode portions (210, 220, 230, 240) include a first RF electrode portion (210), a second RF electrode portion (220), a third RF electrode portion (230), and a fourth RF electrode portion (240) positioned in a line, and a power control portion (300) may apply electrical power to the first RF electrode portion (210) and the fourth RF electrode portion (240) positioned at opposite ends, or may apply electrical power to the second RF electrode portion (220) and the third RF electrode portion (230) positioned between the first RF electrode portion (210) and the fourth RF electrode portion (240), thereby controlling a depth to which RF energy is delivered into the skin (10).

The power control portion (300) may apply electrical power to the first RF electrode portion (210) and the fourth RF electrode portion (240) positioned at the opposite ends to increase a delivery depth of RF energy in the skin (10), or may apply electrical power to the second RF electrode portion (220) and the third RF electrode portion (230) positioned therebetween to decrease the delivery depth of the RF energy in the skin (10).

The power control portion (300) may apply electrical power to the first RF electrode portion (210) and the fourth RF electrode portion (240) positioned at the opposite ends, and apply electrical power to the second RF electrode portion (220) and the third RF electrode portion (230), thereby simultaneously delivering two RF energies to different depths in the skin (10).

That is, the power control portion (300) may simultaneously apply electrical power respectively to the first RF electrode portion (210) and the fourth RF electrode portion (240), and to the second RF electrode portion (220) and the third RF electrode portion (230), or may individually apply electrical power thereto.

In addition, when the power control portion (300) simultaneously applies electrical power to the first RF electrode portion (210) and the fourth RF electrode portion (240), and to the second RF electrode portion (220) and the third RF electrode portion (230), the power control portion (300) may make a voltage of the electrical power applied to the first RF electrode portion (210) and the fourth RF electrode portion (240) different from a voltage of the electrical power applied to the second RF electrode portion (220) and the third RF electrode portion (230).

As one example, the voltage of the electrical power applied to the first RF electrode portion (210) and the fourth RF electrode portion (240) may be greater than the voltage of the electrical power applied to the second RF electrode portion (220) and the third RF electrode portion (230). Alternatively, the voltage of the electrical power applied to the first RF electrode portion (210) and the fourth RF electrode portion (240) may be lower than the voltage of the electrical power applied to the second RF electrode portion (220) and the third RF electrode portion (230).

Accordingly, a difference between a depth of RF energy delivered to the skin through the first RF electrode portion (210) and the fourth RF electrode portion (240) and a depth of RF energy delivered to the skin through the second RF electrode portion (220) and the third RF electrode portion (230) can be more precisely controlled, and thus procedures can be performed under various conditions.

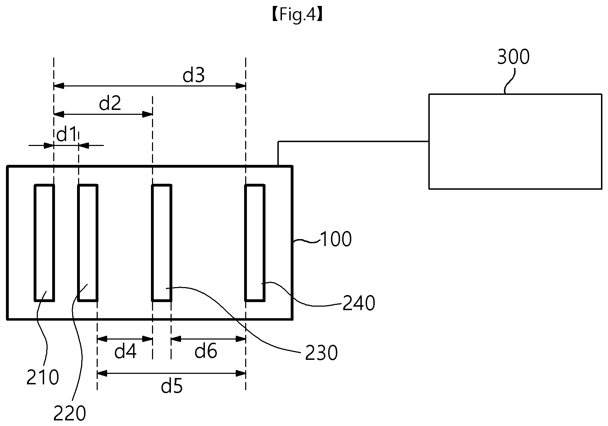

Meanwhile, FIG. 4 is a plan view illustrating still another embodiment of the bipolar RF energy generation device according to the present disclosure, and illustrates one surface of the cartridge housing portion (100), on which at least three RF electrode portions are positioned.

Referring to FIG. 4, four RF electrode portions (210, 220, 230, 240), that is, a first RF electrode portion (210), a second RF electrode portion (220), a third RF electrode portion (230), and a fourth RF electrode portion (240), spaced apart from one another at different intervals may be provided on one surface of the cartridge housing.

The first RF electrode portion (210), the second RF electrode portion (220), the third RF electrode portion (230), and the fourth RF electrode portion (240) are positioned in a line and are spaced apart from one another at different intervals.

An interval between the first RF electrode portion (210) and the second RF electrode portion (220), an interval between the second RF electrode portion (220) and the third RF electrode portion (230), and an interval between the third RF electrode portion (230) and a fourth RF electrode portion (240) are different from one another.

Accordingly, the first RF electrode portion (210) is spaced apart from the second RF electrode portion (220) by a first interval (d₁), spaced apart from the third RF electrode portion (230) by a second interval (d₂), and spaced apart from the fourth RF electrode portion (240) by a third interval (d₃), the second RF electrode portion (220) is spaced apart from the third RF electrode portion (230) by a fourth interval (d₄) and spaced apart from the fourth RF electrode portion (240) by a fifth interval (d5), and the third RF electrode portion (230) is spaced apart from the fourth RF electrode portion (240) by a sixth interval (d6).

The power control portion (300) may select, from among the first RF electrode portion (210), the second RF electrode portion (220), the third RF electrode portion (230), and the fourth RF electrode portion (240), two RF electrode portions spaced apart from each other by any one of the first interval (d1) to the sixth interval (d6), and may apply electrical power thereto, thereby controlling a depth of RF energy delivered to the skin (10) in six stages.

In addition, the power control portion (300) may simultaneously apply electrical power to two pairs of RF electrode portions among the first RF electrode portion (210), the second RF electrode portion (220), the third RF electrode portion (230), and the fourth RF electrode portion (240), thereby simultaneously delivering two RF energies to different depths in the skin (10).

That is, the at least three RF electrode portions (210, 220, 230, 240) are positioned to be spaced apart from one another at different intervals, and the power control portion (300) selects two RF electrode portions among the at least three RF electrode portions (210, 220, 230, 240) and applies electrical power thereto, thereby controlling a depth of RF energy delivered to the skin (10). In addition, by controlling RF energy delivered from the two RF electrode portions, that is, an intensity of an electric field, the power control portion (300) may more precisely and variously control a depth to which the RF energy is delivered into the skin (10).

In addition, the at least three RF electrode portions (210, 220, 230, 240) comprise an even number of at least four RF electrode portions spaced apart from one another, and the power control portion (300) may simultaneously apply electrical power to at least two pairs of RF electrode portions among the even number of at least four RF electrode portions (210, 220, 230, 240), thereby simultaneously delivering a plurality of RF energies to different depths. In addition, by controlling RF energy delivered from the two RF electrode portions, that is, an intensity of an electric field, the power control portion (300) may more precisely and variously control a depth to which each RF energy is delivered into the skin (10).

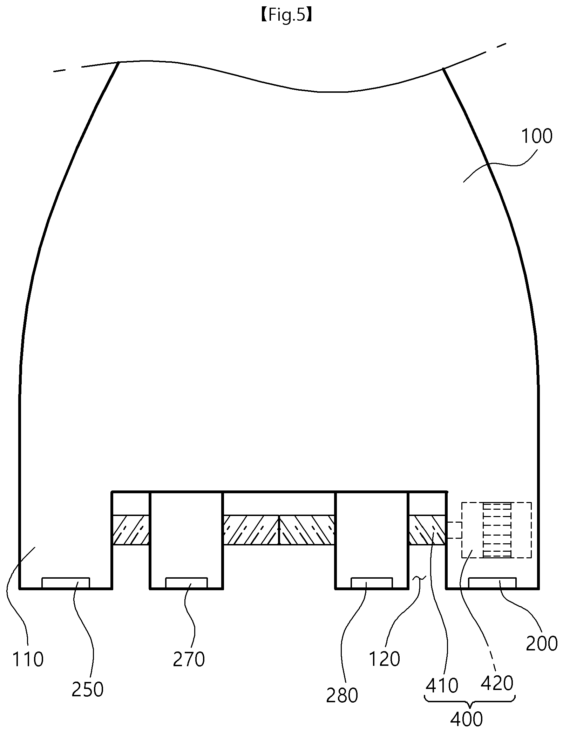

FIG. 5 is a schematic diagram illustrating yet another embodiment of the bipolar RF energy generation device according to the present disclosure. Referring to FIG. 5, the at least three RF electrode portions include at least one fixed electrode portion (250, 260) fixed to one surface of the cartridge housing portion (100) and a plurality of movable electrode portions (270, 280) movably positioned at one side of the fixed electrode portion, and according to yet another embodiment of the present disclosure, the bipolar RF energy generation device may further include an electrode movement portion (400) configured to move the plurality of movable electrode portions (270, 280) toward one side of the fixed electrode portion (250, 260) to adjust an interval between electrodes.

The electrode movement portion (400) may move the plurality of movable electrode portions (270, 280) in a direction spaced apart from the fixed electrode portion (250, 260) to adjust an interval between the fixed electrode portion (250, 260) and the movable electrode portions (270, 280), and the power control portion (300) may apply electrical power to any one of the movable electrode portions (270, 280) in a state in which electrical power is applied to the fixed electrode portion (250, 260), thereby controlling a depth to which RF energy is delivered into the skin (10).

More specifically, according to yet another embodiment of the present disclosure, the bipolar RF energy generation device includes a first fixed electrode portion (250), a second fixed electrode portion (260) positioned at one side of the first fixed electrode portion (250) to be spaced apart therefrom, and a first movable electrode portion (270) and a second movable electrode portion (280) movably positioned between the first fixed electrode portion (250) and the second fixed electrode portion (260).

In addition, the electrode movement portion (400) may move the first movable electrode portion (270) and the second movable electrode portion (280) inward to reduce an interval therebetween, or may move the first movable electrode portion (270) and the second movable electrode portion (280) outward to increase the interval therebetween.

The electrode movement portion (400) may include a movement screw (410) rotatably positioned in the cartridge housing portion (100) and passing through the first movable electrode portion (270) and the second movable electrode portion (280), the first movable electrode portion (270) and the second movable electrode portion (280) being screw-coupled to the movement screw (410) in opposite screw directions, and a screw rotation motor (420) configured to rotate the movement screw (410).

The cartridge housing portion (100) includes, on one surface thereof, an electrode-mounting protrusion (110) provided with the first fixed electrode portion (250) and the second fixed electrode portion (260), and an electrode movement space (120) provided within the electrode-mounting protrusion (110), the first movable electrode portion (270) and the second movable electrode portion (280) being movably positioned in the electrode movement space (120).

As one example, the movement screw (410) is rotatably positioned across the electrode movement space (120), and the screw rotation motor (420) is mounted in the electrode-mounting protrusion (110) and rotates the movement screw (410).

As one example, the movement screw (410) is screw-coupled to the first movable electrode portion (270) in a left-hand screw direction and screw-coupled to the second movable electrode portion (280) in a right-hand screw direction, and it should be noted that the movement screw (410) may be screw-coupled thereto in opposite screw directions.

The first movable electrode portion (270) and the second movable electrode portion (280) are screw-coupled to the movement screw (410) in opposite screw directions, and may be moved inward toward each other or outward away from each other by rotation of the movement screw (410), thereby reducing or increasing an interval therebetween.

The first movable electrode portion (270) and the second movable electrode portion (280) move inward toward each other or outward away from each other between the first fixed electrode portion (250) and the second fixed electrode portion (260), so that not only an interval between the first movable electrode portion (270) and the second movable electrode portion (280), but also an interval between the first fixed electrode portion (250) and the first movable electrode portion (270) and an interval between the second fixed electrode portion (260) and the second movable electrode portion (280) may be simultaneously adjusted.

The power control portion (300) controls operation of the electrode movement portion (400) to adjust an interval between two RF electrode portions to which power is applied, thereby controlling a depth of RF energy delivered to the skin (10). In addition, by controlling RF energy delivered from the two RF electrode portions, that is, an intensity of an electric field, the power control portion (300) may more precisely and variously control a depth to which the RF energy is delivered into the skin (10).

According to the present disclosure, electrical power is selectively applied to two RF electrode portions among at least three RF electrode portions, and an interval between the RF electrode portions to which the electrical power is applied and a frequency of RF energy are controlled, thereby variously controlling a depth to which the RF energy is delivered into the skin (10) and delivering uniform energy according to a depth of the skin (10), so that effects of a procedure can be improved in a bipolar method and types of procedures can be diversified.

Although the present disclosure has been described above in detail using preferred embodiments, the scope of the present disclosure is not limited to the specific embodiments and should be interpreted by the appended claims. In addition, those skilled in the art should understand that many modifications and variations are possible without departing from the scope of the present disclosure.

## Claims

1. A bipolar RF energy generation device comprising:
a cartridge housing portion having one surface configured to contact skin;
at least three RF electrode portions positioned on the one surface of the cartridge housing portion to be spaced apart from one another and configured to contact the skin; and
a power control portion configured to apply electrical power to two of the RF electrode portions and to control a depth of RF energy delivered into the skin by varying a distance between the two RF electrode portions to which the electrical power is applied.

2. The bipolar RF energy generation device of claim 1, wherein the power control portion is configured to apply electrical power to two RF electrode portions selected from among the three RF electrode portions and spaced apart from each other by a first distance, or to apply electrical power to two RF electrode portions selected from among the three RF electrode portions and spaced apart from each other by a second distance longer than the first distance.

3. The bipolar RF energy generation device of claim 1, wherein the at least three RF electrode portions comprise:
a reference RF electrode portion; and
a plurality of selectable RF electrode portions spaced apart from the reference RF electrode portion by different distances,
wherein, when applying electrical power to two electrodes, the power control portion is configured to constantly apply electrical power to the reference RF electrode portion, connect any one of the plurality of selectable RF electrode portions to a power supply line through a power application switch portion, and apply electrical power to the connected selectable RF electrode portion.

4. The bipolar RF energy generation device of claim 2 or claim 3, wherein the at least three RF electrode portions are arranged in a line to be spaced apart from one another.

5. The bipolar RF energy generation device of claim 1, wherein the at least three RF electrode portions comprise a first RF electrode portion, a second RF electrode portion, a third RF electrode portion, and a fourth RF electrode portion positioned in a line, and
the power control portion is configured to apply electrical power to the first RF electrode portion and the fourth RF electrode portion positioned at opposite ends, or to apply electrical power to the second RF electrode portion and the third RF electrode portion positioned between the first RF electrode portion and the fourth RF electrode portion.

6. The bipolar RF energy generation device of claim 1, wherein the at least three RF electrode portions are positioned to be spaced apart from one another at different intervals, and
the power control portion is configured to select two RF electrode portions from among the three RF electrode portions and apply electrical power thereto.

7. The bipolar RF energy generation device of claim 1, wherein the RF electrode portions comprise an even number of at least four RF electrode portions spaced apart from one another, and
the power control portion is configured to simultaneously apply electrical power to at least two pairs of the RF electrode portions.

8. The bipolar RF energy generation device of claim 7, wherein the power control portion is configured to make a voltage of electrical power applied to one pair of the RF electrode portions different from a voltage of electrical power applied to another pair of the RF electrode portions.

9. The bipolar RF energy generation device of claim 1, wherein the at least three RF electrode portions comprise:
at least one fixed electrode portion fixed to the one surface of the cartridge housing portion; and
a plurality of movable electrode portions movably positioned at one side of the fixed electrode portion,
wherein the plurality of movable electrode portions are moved toward one side of the fixed electrode portion by an electrode movement portion provided in the cartridge housing portion.

10. The bipolar RF energy generation device of claim 9, wherein the fixed electrode portion comprises a first fixed electrode portion and a second fixed electrode portion positioned at one side of the first fixed electrode portion to be spaced apart therefrom, and
the movable electrode portions comprise a first movable electrode portion and a second movable electrode portion movably positioned between the first fixed electrode portion and the second fixed electrode portion.

11. The bipolar RF energy generation device of claim 10, wherein the electrode movement portion comprises:
a movement screw rotatably positioned in the cartridge housing portion and passing through the first movable electrode portion and the second movable electrode portion, the first movable electrode portion and the second movable electrode portion being screw-coupled to the movement screw in opposite screw directions; and
a screw rotation motor configured to rotate the movement screw.

12. The bipolar RF energy generation device of claim 11, wherein the cartridge housing portion comprises, on one surface thereof, an electrode-mounting protrusion provided with the first fixed electrode portion and the second fixed electrode portion,
wherein an electrode movement space in which the first movable electrode portion and the second movable electrode portion are movably positioned is provided within the electrode-mounting protrusion.
